# EUROPEAN PATENT SPECIFICATION

(11) **EP 4 258 983 B1**
(45) Date of publication and mention of the grant of the patent: **03.09.2025**
(21) Application number: 21823608.1
(22) Date of filing: 10.12.2021
(51) Int. Cl.: A61B 5/024, A61B 5/11, A61B 5/00, A61H 31/00

(54) **CARDIOPULMONARY RESUSCITATION DECISION SUPPORT**
ENTSCHEIDUNGSUNTERSTÜTZUNG FÜR KARDIOPULMONÄRE WIEDERBELEBUNG
AIDE À LA DÉCISION EN RÉANIMATION CARDIOPULMONAIRE

(30) Priority: 11.12.2020 EP 20213322
(43) Date of publication of application: 18.10.2023
(73) Proprietor: Koninklijke Philips N.V., 5656 AG Eindhoven (NL)
(72) Inventor: MUEHLSTEFF, Jens, 5656 AG Eindhoven (NL); WIJSHOFF, Ralph Wilhelm Christianus Gemma Rosa, 5656 AG Eindhoven (NL); VAN DE LAAR, Jakob, 5656 AG Eindhoven (NL); VEENSTRA, Hugo, 5656 AG Eindhoven (NL); JANSSEN, Anthonius Petrus Gerardus Emanuel, 5656 AG Eindhoven (NL)
(74) Representative: Philips Intellectual Property & Standards
(86) International application number: PCT/EP2021/085131
(87) International publication number: WO 2022/122991

(56) References cited:
- EP-A1- 3 556 281
- WO-A1-2020/011982
- US-A1- 2016 206 504
- US-A1- 2017 281 462
- US-A1- 2018 125 375
- US-A1- 2019 344 068

## Description

### FIELD OF THE INVENTION

The present disclosure relates to a system for providing cardiopulmonary resuscitation (CPR) decision support and a method for operating thereof.

### BACKGROUND OF THE INVENTION

During cardiopulmonary resuscitation (CPR), pulse checks are mainly performed by manual palpation. Palpation requires interruption of the chest compressions, and is a subjective, challenging, and time-consuming procedure. As a result, manual palpation can interrupt the chest compressions for longer than the 10 second duration as recommended by medical guidelines. Palpations tend to be lengthy and time-consuming, especially in the case of potentially perfusing electrocardiography (ECG) rhythms. These long interruptions can negatively impact the outcome of the CPR.

There are some currently available objective methods for checking the status of the circulation during CPR. For example, via invasive arterial blood pressure (iABP) measurements, one can directly see when the heart resumes beating and obtain a quantified measure of the status of the circulation and also during compressions. However, iABP measurements are invasive and therefore they are not common practice during CPR. Alternatively, waveform capnography is a less invasive objective method that provides information about the status of the circulation. Nevertheless, capnography still requires intubation, the interpretation of the end-tidal CO2 level requires well-controlled ventilations and it does not provide any measure of the spontaneous pulse rate (PR). US 2018125375 A1 discloses a system for providing cardiopulmonary resuscitation decision support according to the preamble of claim 1. US 2016206504 A1 discloses another system for providing cardiopulmonary resuscitation decision support.

### SUMMARY OF THE INVENTION

The present invention is directed to a system for providing cardiopulmonary resuscitation decision support according to claim 1. Further aspects of the invention are defined in the dependent claims.

As noted above, there are a number of disadvantages associated with currently available methods for checking the status of the circulation during compression therapy. Furthermore, the pulse oximetry socket of some currently available systems does not support intended application scenarios of CPR, as such applications would require an internal signal fusion along with significant changes to the firmware in the monitors. Also, the measurement sockets at currently available monitors do not facilitate access for additional sensor signals (e.g. accelerometer, environmental light conditions, etc.) with the required synchronicity in signal acquisition. To date, there is no method for supporting pulse detection during CPR which is objective, non-invasive and easy-to-use, and which offers flexibility in the use of reference signals corresponding to compressions.

In the present disclosure, motion-robust photoplethysmography (PPG) is employed for the purpose of pulse determination during compression therapy. Preclinical and clinical data have demonstrated good potential of using PPG for detecting a spontaneous pulse during pauses and ongoing compressions during CPR. Furthermore, algorithms have been developed for automated detection of a spontaneous pulse in a PPG signal during CPR. For example, international patent application WO 2017/211814 describes a technique by which a PPG signal can be used to detect a return (or continued presence) of a spontaneous pulse during the application of CPR. These algorithms rely on frequency analysis and require a reference signal to handle artefacts caused by compressions so as to reveal the presence of a potentially existing spontaneous pulse signal. Currently, the trans-thoracic impedance signal from a defibrillator or a monitor is used as a reference signal for the chest compressions. However, the need for a full integration of such algorithms in a monitor (or a defibrillator) limits the applicability of the approach.

Nevertheless, one of the ways to realize PPG as a way to detect a spontaneous pulse during compression therapy is to implement essential system components external of the monitor or the defibrillator so as to facilitate basic functionalities. This concept is sometimes referred to as "Smart Cables" or "Smart Measurements". In some currently available systems, measurement hardware is integrated into cables that are connected by a standardized interface. In these systems, the monitor could be the user interface, but it could also be the processing unit (in addition).

It would therefore be advantageous to provide an improved system for enabling CPR decision support based on motion-robust low-perfusion pulse oximetry as a standalone monitoring tool that supports both manual CPR and automated CPR.

According to a first specific aspect, there is provided a system for providing cardiopulmonary resuscitation (CPR) decision support, the system comprising: a photoplethysmography (PPG) sensing unit configured to determine one or more PPG signals at a measurement site on a subject, a core unit comprising a user interface, a motion sensing unit configured to detect motions correlated to chest compressions during compression therapy on the subject, and a processing unit configured to determine presence or absence of a spontaneous pulse based on the detected motions correlated to chest compressions during compression therapy on the subject and the one or more PPG signals, determine a recommendation to be provided based on the determination of presence or absence of a spontaneous pulse, wherein the recommendation is associated with CPR decision support, and control the user interface to output the determined recommendation.

In some embodiments, the motion sensing unit may be part of the core unit.

In some embodiments, if it is determined that a spontaneous pulse is present, the determined recommendation may be to perform a further check for presence of a pulse and/or to withhold administration of a vasopressor.

In some embodiments, if it is determined that a spontaneous pulse is absent, the determined recommendation may be to continue compression therapy on the subject.

In some embodiments, the PPG sensing unit may comprise at least one of: a nasal alar PPG sensor, a nasal columella PPG sensor, an ear concha PPG sensor, and a forehead PPG sensor. In these embodiments, the PPG sensing unit may comprise more than one of: a nasal alar PPG sensor, a nasal columella PPG sensor, an ear concha PPG sensor, and a forehead PPG sensor, and the user interface may be configured to receive a user input indicating at least one sensor to activate for determining the one or more PPG signals. Alternatively or in addition, the processing unit may receive signal quality indicator(s) corresponding to one or more of the PPG sensors, and one or more PPG sensors may be activated based on the received signal quality indicator(s). Alternatively or in addition, one or more PPG sensors may be activated based on the received user input and the received signal quality indicator(s).

In some embodiments, the processing unit may be further configured to: acquire a core body temperature value of the subject, and control the user interface to output at least one of: the detected core body temperature, an indication of whether the detected core body temperature is within a predetermined target range or at a predetermined target value, and an indication of whether the detected core body temperature is approaching a predetermined target range or a predetermined target value.

In some embodiments, the processing unit may be further configured to acquire at least one of: an electrocardiography (ECG) signal associated with the subject and a signal indicating nasal flow of the subject. In these embodiments, determining the recommendation to be provided may be further based on the acquired at least one of an ECG signal and a signal indicating nasal flow of the subject.

In some embodiments, the processing unit may be further configured to: determine whether amplitudes and/or a signal quality corresponding to the detected motions correlated to chest compressions during compression therapy on the subject are within a predetermined target range, and control the user interface to output at least one of: an indication of the result of the determination of whether the amplitudes and/or the signal quality are within a predetermined target range, and an instruction to adjust a position of the core unit with an integrated motion sensing unit or the motion sensing unit.

In some embodiments, the processing unit may be implemented at a mobile device, and the processing unit may be configured to connect wirelessly to at least one of: the core unit, the motion sensing unit, and the PPG sensing unit.

In some embodiments, the processing unit may be part of the core unit, and the core unit may be configured such that it can be placed at or adjacent to an upper part of the chest area of the subject.

In some embodiments, the core unit may be implemented at a mobile device.

In some embodiments, the mobile device may be a smartphone.

According to a second specific aspect, there is provided a method for operating a system for providing cardiopulmonary resuscitation (CPR) decision support, wherein the system comprises a photoplethysmography (PPG) sensing unit, a core unit comprising a user interface, a motion sensing unit, and a processing unit, the method comprising: determining, at the PPG sensing unit, one or more PPG signals at a measurement site on a subject; detecting, at the motion sensing unit, motions correlated to chest compressions during compression therapy on the subject; determining, at the processing unit, presence or absence of a spontaneous pulse based on the detected motions correlated to chest compressions during compression therapy on the subject and the one or more PPG signals; determining, at the processing unit, a recommendation to be provided based on the determination of presence or absence of a spontaneous pulse; and controlling, at the processing unit, the user interface to output the determined recommendation.

According to aspects and embodiments as described above, the limitations of existing techniques are addressed. In particular, at least some of the above-described aspects and embodiments provide a standalone "Smart Cable" solution that is cost-effective compared to a fully integrated PPG measurement approach in a monitor or a defibrillator. Some aspects and embodiments also offer easy compatibility with third party systems (e.g. when used with a CPR robot, compressions would not be interrupted if no pulse is present, and compressions can be stopped according to a determined recommendation when pulse presence is detected). Moreover, some aspects and embodiments allow the required measurements for CPR-proof pulse oximetry to be used as reference for the detection of a spontaneous pulse, while offering flexibility in the use of various different signals corresponding to compressions. In addition, since at least some of the aspects and embodiments adopt both wired and wireless transmission techniques and modes, clutter issues in particular during cardiopulmonary resuscitation can be reduced or avoided.

These and other aspects will be apparent from and elucidated with reference to the embodiment(s) described hereinafter.

### BRIEF DESCRIPTION OF THE DRAWINGS

Exemplary embodiments will now be described, by way of example only, with reference to the following drawings, in which:
Fig. 1 is a block diagram of a system for providing cardiopulmonary resuscitation (CPR) decision support, according to an embodiment; and
Fig. 2 is a flow chart for a method for operating a system for providing cardiopulmonary resuscitation (CPR) decision support, according to an embodiment.

### DETAILED DESCRIPTION OF EMBODIMENTS

As noted above, there is provided an improved system and a method of operating the same, which address the existing problems.

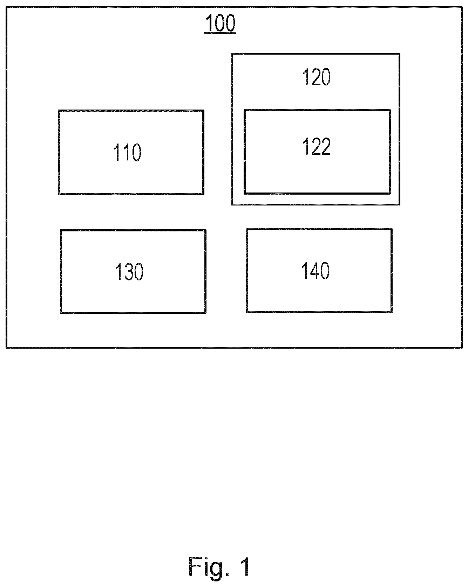

Fig. 1 is a block diagram of a system 100 for providing cardiopulmonary resuscitation (CPR) decision support. The system 100 does not require integration into a patient monitor or a defibrillator in order to perform the functions as described below, and it can be used in scenarios such as out-of-hospital CPR.

As shown in Fig. 1, the system 100 comprises a photoplethysmography (PPG) sensing unit 110, a core unit 120, a motion sensing unit 130, and a processing unit 140. The connections between the components of the system 100, particularly between the core unit 120 and the other components and between the processing unit 140 and the other components, can be implemented wirelessly, e.g. Global System for Mobile Communications (GSM), Bluetooth, Bluetooth Low Energy, and/or Near-field Communication (NFC). In some embodiments, the connection between the PPG sensing unit 110 and the core unit 120 may be implemented via a digital signal interface, as an example. Furthermore, in some embodiments at least some connections between the components of the system 100 can be switchable between wired and wireless.

The PPG sensing unit 110 is configured to determine one or more PPG signals at a measurement site on a subject. The PPG sensing unit 110 may comprise a low-perfusion oximetry sensor, and more specifically the PPG sensing unit 110 may comprise at least one of: a nasal alar PPG sensor, a nasal columella PPG sensor, an ear concha PPG sensor, and a forehead PPG sensor.

The core unit 120 further comprises a user interface 122. The user interface 122 may be for use in providing a user of the system 100 with information resulting from the techniques described herein. Alternatively or in addition, the user interface 122 may be configured to receive a user input. For example, the user interface 122 may allow a user of the system 100 to manually enter instructions, data, or information.

The user interface 122 may be any user interface that enables the rendering (or output or display) of information to a user of the system 100. Alternatively or in addition, the user interface 122 may be any user interface that enables a user of the system 100 to provide a user input, interact with and/or control the system 100. For example, the user interface 122 may comprise one or more switches, one or more buttons, a keypad, a keyboard, a touch screen or an application (for example, on a tablet or smartphone), a display screen, a graphical user interface (GUI) or other visual rendering component, one or more speakers, one or more microphones or any other audio component, one or more lights, a component for providing tactile feedback (e.g. a vibration function), or any other user interface, or combination of user interfaces.

As mentioned above, in some embodiments the PPG sensing unit 110 may comprise at least one of: a nasal alar PPG sensor, a nasal columella PPG sensor, an ear concha PPG sensor, and a forehead PPG sensor. In these embodiments, the PPG sensing unit 110 may comprise more than one of: a nasal alar PPG sensor, a nasal columella PPG sensor, an ear concha PPG sensor, and a forehead PPG sensor, and the user interface 122 may be configured to receive a user input indicating at least one sensor to activate for determining the one or more PPG signals. Alternatively or in addition, the processing unit 140 may receive signal quality indicator(s) corresponding to one or more of the PPG sensors, and one or more PPG sensors may be activated based on the received signal quality indicator(s) under the control of the processing unit 140 (i.e. the processing unit 140 can determine at least one of the one or more PPG sensors to be activated based on the received signal quality indicator(s)). Alternatively or in addition, one or more PPG sensors may be activated based on the received user input and the received signal quality indicator(s). Therefore, user(s) can decide which sensor(s) to use based on, for example, best compatibility with the user's workflow. For example, this decision may be based on whether ventilation of the subject is performed using a mask or via intubation.

The motion sensing unit 130 is configured to detect motions correlated to chest compressions during compression therapy on the subject. In some embodiments, the motion sensing unit 130 may be a part of the core unit 120. The motion sensing unit 130 may comprise one or more accelerometers.

The processing unit 140 is configured to determine presence or absence of a spontaneous pulse based on the detected motions correlated to chest compressions during compression therapy on the subject (from the motion sensing unit 130), and the one or more PPG signals (from the PPG sensing unit 110). For example, accelerometry traces of the detected motions can be used as a reference of chest compressions, such that the effects of compression therapy can be taken into account in the determination of presence or absence of a spontaneous pulse. In some embodiments, this determination may be performed according to one or more algorithms developed for the purpose of detecting a return (or continued presence) of a spontaneous pulse during the application of CPR, such as the technique described in international patent application WO 2017 /211814, which includes a method that results in the output of a result of a classified pulse condition ("no pulse, "pulse", and "indeterminate"), the detection being based on compressions reference signals and PPG signals.

The processing unit 140 is further configured to determine a recommendation to be provided based on the determination of presence or absence of a spontaneous pulse, the recommendation being associated with CPR decision support. For example, if it is determined by the processing unit 140 that a spontaneous pulse is present, the recommendation may be to perform a further check for presence of a pulse and/or withhold administration of a vasopressor, and/or if it is determined by the processing unit 140 that a spontaneous pulse is absent, the recommendation may be to continue compression therapy on the subject.

Since the recommendation is determined based on presence or absence of a spontaneous pulse, this recommendation enables patient safety to be improved especially when using automated CPR techniques (e.g. CPR robot), because recommendations such as "further check for presence of a pulse" can be provided which prompts a user to check for pulse presence before stopping or continuing CPR therapy. Moreover, the determined recommendation enables patient care outcome to be improved, since unnecessary pulse checks and the associated interruptions in compression therapy can be avoided.

The processing unit 140 is further configured to control the user interface 122 to output the determined recommendation, e.g. via a display screen of the user interface 122. As a more specific example, the output may involve outputting, via a display screen of the user interface 122, text information which reads "perform a further check for presence of a pulse" or "withhold administration of a vasopressor" or "continue CPR".

In some embodiments, one or more other key basic vital signs (e.g. SpO2, pulse rate, core body temperature, and respiration rate) can be monitored by the system 100 to provide further information on the patient condition or to provide further advice during CPR therapy or after return of spontaneous circulation (ROSC). For example, in some embodiments, the processing unit 140 may be further configured to acquire a core body temperature (CBT) value of the subject, and to control the user interface 122 to output at least one of: the detected core body temperature, an indication of whether the detected core body temperature is within a predetermined target range or at a predetermined target value, and an indication of whether the detected core body temperature is approaching a predetermined target range or a predetermined target value. Since during CPR a controlled decrease and/or maintenance of appropriate CBT is important to the outcome of CPR (e.g. so as to preserve brain function), by indicating the CBT (and/or whether it is within a target range or at a target value) via the user interface 122, user(s) can be provided with useful information during CPR which can help achieve a desired outcome more efficiently and effectively.

For this purpose, the system 100 may further comprise a core body temperature (CBT) sensing unit, the CBT sensing unit being configured to detect the core body temperature of the subject. Accordingly, in these embodiments, the CBT value of the subject may be acquired by the processing unit 140 from the CBT sensing unit. Moreover, in some embodiments, the CBT sensing unit may be a part of the core unit 120. Alternatively, the CBT value of the subject may be provided by a CBT sensing unit that is external to the system 100.

In some embodiments, the processing unit 140 may be further configured to acquire at least one of: an electrocardiography (ECG) signal associated with the subject and a signal indicating nasal flow of the subject. In these embodiments, the processing unit 140 may be configured to determine the recommendation to be provided further based on the acquired at least one of an ECG signal and a signal indicating nasal flow of the subject. For example, a recommendation (e.g. whether and/or when to start or restart compression therapy, or follow-up action(s) after return of spontaneous circulation (ROSC)) can be provided based on a respiration rate of the subject that is derived, at the processing unit 140 or otherwise, from the signal indicating nasal flow of the subject. As another example, the ECG signal may be used by the processing unit 140 to confirm organized electrical activity of the heart of the subject, which is a prerequisite for pulse presence.

In some embodiments the processing unit 140 may be configured to determine presence or absence of a spontaneous pulse further based on the acquired at least one of an ECG signal and a signal indicating nasal flow of the subject, and thereby a recommendation can be provided based on the presence or the absence of a spontaneous pulse.

In some embodiments, the system 100 may further comprise an ECG sensing unit which is configured to detect an ECG signal associated with the subject. In these embodiments, the ECG signal may be acquired by the processing unit 140 from the ECG sensing unit. The ECG sensing unit may comprise, for example, ECG electrodes to be placed at skin contact measurement sites (e.g. the nose or at the core unit 120) on the subject. Alternatively, the ECG signal may be acquired from an ECG sensing unit external to the system 100.

In some embodiments, the system 100 may further comprise a nasal flow sensing unit configured to detect a signal indicating nasal flow of the subject. In these embodiments, the signal indicating nasal flow of the subject may be acquired by the processing unit 140 from the nasal flow sensing unit. Alternatively, the signal indicating nasal flow of the subject may be acquired from a nasal flow sensing unit external to the system 100.

In some embodiments, the processing unit 140 may be further configured to: determine whether amplitudes and/or a signal quality corresponding to the detected motions correlated to chest compressions during compression therapy on the subject are within a predetermined target range, and control the user interface to output at least one of: an indication of the result of the determination of whether the amplitudes and/or the signal quality are within a predetermined target range (e.g. a warning that the signal quality is not sufficient enough to allow compression-induced oscillations in the detected motions to be discerned, or not sufficient enough for respective algorithm(s) for the purpose of spontaneous pulse presence detection), and an instruction to adjust a position of the core unit 120 with an integrated motion sensing unit or the motion sensing unit 130 itself. The instruction to adjust a position of the core unit 120 with the integrated motion sensing unit or the motion sensing unit 130 itself may provide information that can facilitate or guide user(s) in adjusting the position of the core unit 120 with the integrated motion sensing unit or the motion sensing unit 130 such that sufficient and/or accurate motion sensing corresponding to compression-induced movements can be achieved.

In some embodiments, the processing unit 140 may be implemented at a mobile device, and the processing unit is configured to connect wirelessly to at least one of: the core unit 120, the motion sensing unit 130, and the PPG sensing unit 110. In these embodiments, raw or filtered PPG signal(s) may be transmitted to the processing unit 140 via a wired or a wireless connection, for example directly from the PPG sensing unit 110 or via the core unit 120. As mentioned above, in some embodiments the determination of presence or absence of a spontaneous pulse may be performed at the processing unit 140 according to one or more algorithms developed for the purpose of detecting a return (or continued presence) of a spontaneous pulse during the application of CPR. In these embodiments, the relevant algorithm(s) may be run at the mobile device, e.g. using a mobile application installed in the mobile device. By running the determination algorithm(s) using a mobile application installed in a mobile device, updates for the algorithm(s) can be more conveniently and effectively rolled out.

In some embodiments, the processing unit 140 may be part of the core unit 120, and the core unit 120 may be configured such that it can be placed at or adjacent to an upper part of the chest area of the subject. The placement of the core unit 120 at or adjacent to an upper part of the chest area of the subject ensures that it does not interfere with ongoing compression therapy on the subject. Furthermore, in some embodiments, motion sensing unit 130 may be part of the core unit 120, in which case placement of the core unit 120 at or adjacent to an upper part of the chest area of the subject ensures adequate measurement of the motions correlated to the chest compressions during compression therapy on the subject. Moreover, in these embodiments, the core unit may be implemented at a mobile device (e.g. a smartphone or a tablet). Specifically, in some implementations the motion sensing unit 130 may be a part of the core unit 120 which may be an embedded component (e.g. accelerometer) of the mobile device.

Although not shown in the drawing, in some embodiments the system 100 may further comprise a communications interface (or circuitry) for enabling the system 100 to communicate with any interfaces, memories and/or devices that are internal or external to the system 100. The communications interface may communicate with any interfaces, memories and/or devices wirelessly or via a wired connection. For example, the communications interface may communicate with one or more user interfaces wirelessly or via a wired connection. Similarly, the communications interface may communicate with the one or more memories wirelessly or via a wired connection.

It will be appreciated that Fig. 1 only shows the components required to illustrate an aspect of the system 100 and, in a practical implementation, the system 100 may comprise alternative or additional components to those shown. For example, in some embodiments the system 100 may comprise a power source (e.g. implemented at the core unit 120).

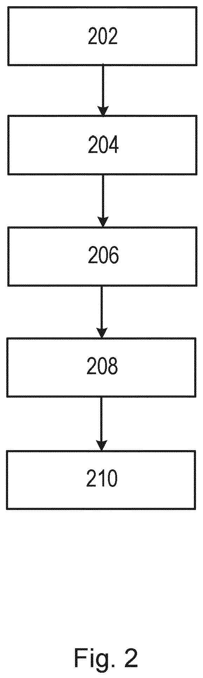

Fig. 2 is a flow chart for a method for operating a system for providing cardiopulmonary resuscitation (CPR) decision support, such as the system 100 as illustrated in Fig. 1. The system comprises a photoplethysmography (PPG) sensing unit, a core unit comprising a user interface, a motion sensing unit, and a processing unit. In order to facilitate understanding, some of the description below will be made with reference to the various components of the system 100 as shown in Fig. 1.

With reference to Fig. 2, at block 202, one or more PPG signals at a measurement site on a subject is determined at the PPG sensing unit 110, which may comprise at least one of: a nasal alar PPG sensor, a nasal columella PPG sensor, an ear concha PPG sensor, and a forehead PPG sensor.

In some embodiments, prior to block 202, the method may further comprise receiving, at the user interface 122 of the system 100, a user input indicating at least one sensor to activate for determining the one or more PPG signals. Alternatively or in addition, the method may comprise receiving, at the processing unit 140, signal quality indicator(s) corresponding to one or more of the sensors, and one or more sensors may be activated based on the received signal quality indicator(s). Alternatively or in addition, the method may comprise activating one or more PPG sensors based on the received user input and the received signal quality indicator(s). Therefore, user(s) can decide which sensor(s) to use based on, for example, best compatibility with the user's workflow. For example, this decision may be based on whether ventilation of the subject is performed using a mask or via intubation.

Returning to Fig. 2, at block 204, motions correlated to chest compressions during compression therapy on the subject are detected at the motion sensing unit 130.

Subsequently, at block 206, presence or absence of a spontaneous pulse is determined, at the processing unit 140, based on the detected motions correlated to chest compressions during compression therapy on the subject and the one or more PPG signals. This determination may be performed according to one or more algorithms developed for the purpose of detecting a return (or continued presence) of a spontaneous pulse during the application of CPR, such as the technique described in international patent application WO 2017 /211814, which includes a method that results in the output of a result of a classified pulse condition ("no pulse, "pulse", and "indeterminate"), the detection being based on compressions reference signals and PPG signals. Furthermore, in some embodiments, the one or more algorithms may be implemented by way of a mobile application installed on a mobile device.

Then, at block 208, a recommendation to be provided is determined at the processing unit 140, based on the determination of presence or absence of a spontaneous pulse. For example, if it is determined at block 206 that a spontaneous pulse is present, the recommendation at block 208 may be to perform a further check for presence of a pulse and/or to withhold administration of a vasopressor. As another example, if it is determined at block 206 that a spontaneous pulse is absent, the recommendation at block 208 may be to continue compression therapy on the subject. As a more specific example, the output may involve outputting, via a display screen of the user interface 122, text information which reads "perform a further check for presence of a pulse" and/or "withhold administration of a vasopressor" or "continue CPR".

In some embodiments, the method may further comprise acquiring, at the processing unit 140, at least one of: an electrocardiography (ECG) signal associated with the subject and a signal indicating nasal flow of the subject. The ECG signal may be acquired from an ECG sensing unit of the system, or an ECG sensing unit external to the system. The signal indicating nasal flow of the subject may be acquired from a nasal flow sensing unit of the system, or a nasal flow sensing unit external to the system. In these embodiments, determining the recommendation to be provided at block 208 may be further based on the acquired at least one of an ECG signal and a signal indicating nasal flow of the subject. For example, a recommendation (e.g. whether and/or when to start or restart compression therapy, or follow-up action(s) after return of spontaneous circulation (ROSC)) can be provided based on a respiration rate of the subject that is derived, at the processing unit 140 or otherwise, from the signal indicating nasal flow of the subject. As another example, the ECG signal may be used by the processing unit 140 to confirm organized electrical activity of the heart of the subject, which is a prerequisite for pulse presence.

In some embodiments, determining presence or absence of a spontaneous pulse at block 206 may be further based on the acquired at least one of an ECG signal and a signal indicating nasal flow of the subject, and thereby a recommendation can be determined at block 208 based on the presence or the absence of a spontaneous pulse.

Returning to Fig. 2, at block 210, the user interface 122 of the core unit 120 is controlled so as to output the recommendation determined at block 208.

In some embodiments, the method may further comprise acquiring, at the processing unit 140, a core body temperature (CBT) value of the subject. The CBT value may be acquired from a CBT sensing unit of the system 100, and/or from a CBT sensing unit that is external to the system 100. In these embodiments, the method may further comprise controlling, at the processing unit 140, the user interface 122 to output at least one of: the detected core body temperature, an indication of whether the detected core body temperature is within a predetermined target range or at a predetermined target value, and an indication of whether the detected core body temperature is approaching a predetermined target range or a predetermined target value. Since during CPR a controlled decrease and/or maintenance of appropriate CBT is important to the outcome of CPR (e.g. so as to preserve brain function), by indicating the CBT (and/or whether it is within a target range or at a target value) by the user interface 122, user(s) can be provided with useful information during CPR which can help achieve a desired outcome more efficiently and effectively.

In some embodiments, the method may further comprise determining, at the processing unit 140, whether amplitudes and/or a signal quality corresponding to the detected motions correlated to chest compressions during compression therapy on the subject are within a predetermined target range, and controlling, at the processing unit 140, the user interface 122 to output at least one of: an indication of the result of the determination of whether the amplitudes and/or the signal quality are within a predetermined target range (e.g. a warning that the signal quality is not sufficient enough to allow compression-induced oscillations in the detected motions to be discerned), and an instruction to adjust a position of the core unit 120 with an integrated motion sensing unit or motion sensing unit 130 itself. The instruction to adjust a position of the core unit 120 with the integrated motion sensing unit or the motion sensing unit 130 itself may provide information for user(s) to adjust the position of the core unit 120 with the integrated motion sensing unit or the motion sensing unit 130 itself such that sufficient and/or accurate motion sensing corresponding to compression-induced movements can be achieved.

There is thus provided an improved system and method for operating the same, which overcomes the existing problems.

There is also provided a computer program product comprising a computer readable medium, the computer readable medium having computer readable code embodied therein, the computer readable code being configured such that, on execution by a suitable computer or processor, the computer or processor is caused to perform the method or methods described herein. Thus, it will be appreciated that the disclosure also applies to computer programs, particularly computer programs on or in a carrier, adapted to put embodiments into practice. The program may be in the form of a source code, an object code, a code intermediate source and an object code such as in a partially compiled form, or in any other form suitable for use in the implementation of the method according to the embodiments described herein.

It will also be appreciated that such a program may have many different architectural designs. For example, a program code implementing the functionality of the method or system may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other.

An embodiment relating to a computer program product comprises computer-executable instructions corresponding to each processing stage of at least one of the methods set forth herein. These instructions may be sub-divided into sub-routines and/or stored in one or more files that may be linked statically or dynamically. Another embodiment relating to a computer program product comprises computer-executable instructions corresponding to each means of at least one of the systems and/or products set forth herein. These instructions may be sub-divided into sub-routines and/or stored in one or more files that may be linked statically or dynamically.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A system (100) for providing cardiopulmonary resuscitation, CPR, decision support, the system comprising:
a photoplethysmography, PPG, sensing unit (110) configured to determine one or more PPG signals at a measurement site on a subject;
a core unit (120) comprising a user interface (122);
a motion sensing unit (130) configured to detect motions correlated to chest compressions during compression therapy on the subject; and
a processing unit (140) configured to:
determine presence or absence of a spontaneous pulse based on the detected motions correlated to chest compressions during compression therapy on the subject and the one or more PPG signals;
determine a recommendation to be provided based on the determination of presence or absence of a spontaneous pulse, wherein the recommendation is associated with CPR decision support;
control the user interface to output the determined recommendation;
**characterised in that** the processing unit is further configured to:
determine whether amplitudes and/or a signal quality corresponding to the
detected motions correlated to chest compressions during compression therapy on the subject are within a predetermined target range;
control the user interface to output an instruction to adjust a position of the motion sensing unit or a position of the core unit if the motion sensing unit is integrated in the core unit, if it is determined that the amplitudes and/or the signal quality corresponding to the detected motions correlated to chest compressions during compression therapy on the subject are not within the predetermined target range; and
guide a user in adjusting the position of the core unit and/or the motion sensing unit by using the reading of the motion sensing unit.

2. The system according to claim 1, wherein the motion sensing unit is part of the core unit.

3. The system according to claim 1 or claim 2, wherein if it is determined that a spontaneous pulse is present, the determined recommendation is to perform a further check for presence of a pulse and/or to withhold administration of a vasopressor.

4. The system according to any one of the preceding claims, wherein if it is determined that a spontaneous pulse is absent, the determined recommendation is to continue compression therapy on the subject.

5. The system according to any one of the preceding claims, wherein the PPG sensing unit comprises at least one of: a nasal alar PPG sensor, a nasal columella PPG sensor, an ear concha PPG sensor, and a forehead PPG sensor.

6. The system according to claim 5, wherein the PPG sensing unit comprises more than one of: a nasal alar PPG sensor, a nasal columella PPG sensor, an ear concha PPG sensor, and a forehead PPG sensor, and the user interface is configured to receive a user input indicating at least one sensor to activate for determining the one or more PPG signals.

7. The system according to claim 5 or claim 6, wherein the PPG sensing unit comprises more than one of: a nasal alar PPG sensor, a nasal columella PPG sensor, an ear concha PPG sensor, and a forehead PPG sensor, and wherein the processing unit is configured to:
receive one or more signal quality indicators corresponding to one or more of the PPG sensors; and
determine at least one of the one or more PPG sensors to activate based on the received one or more signal quality indicators.

8. The system according to any one of the preceding claims, and wherein the processing unit is further configured to:
acquire a core body temperature value of the subject; and
control the user interface to output at least one of: the detected core body temperature, an indication of whether the detected core body temperature is within a predetermined target range or at a predetermined target value, and an indication of whether the detected core body temperature is approaching a predetermined target range or a predetermined target value.

9. The system according to any one of the preceding claims, wherein the processing unit is further configured to acquire at least one of: an electrocardiography, ECG, signal associated with the subject and a signal indicating nasal flow of the subject,
wherein determining the recommendation to be provided is further based on the acquired at least one of an ECG signal and a signal indicating nasal flow of the subject.

10. The system according to any one of the preceding claims, wherein the processing unit is further configured to control the user interface to further output an indication of the result of the determination of whether the amplitudes and/or the signal quality are within a predetermined target range.

11. The system according to any one of the preceding claims, wherein the processing unit is implemented at a mobile device, and the processing unit is configured to connect wirelessly to at least one of: the core unit, the motion sensing unit, and the PPG sensing unit.

12. The system according to any one of claims 1 to 10, wherein the processing unit is part of the core unit, and the core unit is configured such that it can be placed at or adjacent to an upper part of the chest area of the subject.

13. The system according to claim 12, wherein the core unit is implemented at a mobile device.

14. The system according to claim 11 or claim 13, wherein the mobile device is a smartphone.

## Patentansprüche

1. System (100) zur Bereitstellung von Entscheidungsunterstützung für kardiopulmonale Wiederbelebung, CPR, wobei das System umfasst:
eine Photoplethysmographie-, PPG-, Sensoreinheit (110), welche dazu konfiguriert ist, ein oder mehrere PPG-Signale an einer Messstelle an einem Subjekt zu bestimmen;
eine Kerneinheit (120), welche eine Benutzerschnittstelle (122) umfasst;
eine Bewegungserfassungseinheit (130), welche dazu konfiguriert ist, Bewegungen zu erfassen, welche mit Brustkompressionen während Kompressionstherapie an dem Subjekt korrelieren; und
eine Verarbeitungseinheit (140), welche dazu konfiguriert ist:
Vorhandensein oder Fehlen eines spontanen Pulses basierend auf den erfassten Bewegungen zu bestimmen, welche mit Brustkompressionen während einer Kompressionstherapie an dem Subjekt und dem einen oder den mehreren PPG-Signalen korrelieren;
eine Empfehlung zu bestimmen, welche basierend auf der Bestimmung des Vorhandenseins oder Fehlens eines spontanen Pulses bereitgestellt werden soll, wobei die Empfehlung mit CPR-Entscheidungsunterstützung verknüpft ist;
die Benutzerschnittstelle zum Ausgeben der bestimmten Empfehlung zu steuern;
**dadurch gekennzeichnet, dass** die Verarbeitungseinheit weiter dazu konfiguriert ist:
zu bestimmen, ob Amplituden und/oder eine Signalqualität, welche den erfassten Bewegungen entsprechen, welche mit Brustkompressionen während einer Kompressionstherapie an dem Subjekt korrelieren, innerhalb eines vorbestimmten Zielbereichs liegen;
die Benutzerschnittstelle zu steuern, um eine Anweisung zum Anpassen einer Position der Bewegungserfassungseinheit oder einer Position der Kerneinheit, wenn die Bewegungserfassungseinheit in die Kerneinheit integriert ist, auszugeben, wenn bestimmt wird, dass die Amplituden und/oder die Signalqualität, welche den erfassten Bewegungen entsprechen, welche mit Brustkompressionen während einer Kompressionstherapie an dem Subjekt korrelieren, nicht innerhalb des vorbestimmten Zielbereichs liegen; und
einen Benutzer beim Anpassen der Position der Kerneinheit und/oder der Bewegungserfassungseinheit anzuleiten, indem der Messwert der Bewegungserfassungseinheit verwendet wird.

2. System nach Anspruch 1, wobei die Bewegungserfassungseinheit Teil der Kerneinheit ist.

3. System nach Anspruch 1 oder Anspruch 2, wobei, wenn bestimmt wird, dass ein spontaner Puls vorhanden ist, die bestimmte Empfehlung darin besteht, eine weitere Überprüfung auf Vorhandensein eines Pulses durchzuführen und/oder die Verabreichung eines Vasopressors zu unterlassen.

4. System nach einem der vorstehenden Ansprüche, wobei, wenn bestimmt wird, dass kein spontaner Puls vorhanden ist, die bestimmte Empfehlung darin besteht, die Kompressionstherapie des Subjekts fortzusetzen.

5. System nach einem der vorstehenden Ansprüche, wobei die PPG-Sensoreinheit zumindest eines der Folgenden umfasst: einen PPG-Sensor für die Nasenflügel, einen PPG-Sensor für die Nasen-Columella, einen PPG-Sensor für die Ohrmuschel und einen PPG-Sensor für die Stirn.

6. System nach Anspruch 5, wobei die PPG-Sensoreinheit mehr als eines der Folgenden umfasst: einen PPG-Sensor für die Nasenflügel, einen PPG-Sensor für die Nasen-Columella, einen PPG-Sensor für die Ohrmuschel und einen PPG-Sensor für die Stirn, und die Benutzerschnittstelle dazu konfiguriert ist, eine Benutzereingabe zu empfangen, welche zumindest einen Sensor angibt, welcher zum Bestimmen des einen oder der mehreren PPG-Signale aktiviert werden soll.

7. System nach Anspruch 5 oder Anspruch 6, wobei die PPG-Sensoreinheit mehr als eines der Folgenden umfasst: einen PPG-Sensor für die Nasenflügel, einen PPG-Sensor für die Nasen-Columella, einen PPG-Sensor für die Ohrmuschel und einen PPG-Sensor für die Stirn, und wobei die Verarbeitungseinheit dazu konfiguriert ist:
einen oder mehrere Signalqualitätsindikatoren zu empfangen, welche einem oder mehreren der PPG-Sensoren entsprechen; und
zu bestimmen, dass zumindest einer des einen oder der mehreren PPG-Sensoren basierend auf dem einen oder den mehreren empfangenen Signalqualitätsindikatoren aktiviert werden soll.

8. System nach einem der vorstehenden Ansprüche, und wobei die Verarbeitungseinheit weiter dazu konfiguriert ist:
einen Körperkerntemperaturwert des Subjekts zu erfassen; und
die Benutzerschnittstelle zu steuern, um zumindest eines der Folgenden auszugeben: die erfasste Körperkerntemperatur, eine Anzeige, ob die erfasste Körperkerntemperatur innerhalb eines vorbestimmten Zielbereichs oder bei einem vorbestimmten Zielwert liegt, und eine Anzeige, ob die erfasste Körperkerntemperatur sich einem vorbestimmten Zielbereich oder einem vorbestimmten Zielwert nähert.

9. System nach einem der vorstehenden Ansprüche, wobei die Verarbeitungseinheit weiter dazu konfiguriert ist, zumindest eines der Folgenden zu erfassen: ein mit dem Subjekt verknüpftes Elektrokardiographie-, EKG-, Signal und ein Signal, welches den nasalen Atemfluss des Subjekts anzeigt,
wobei Bestimmen der bereitzustellenden Empfehlung weiter auf dem erfassten zumindest einem von einem EKG-Signal und/oder einem Signal, welches den nasalen Atemfluss des Subjekts anzeigt, basiert.

10. System nach einem der vorstehenden Ansprüche, wobei die Verarbeitungseinheit weiter dazu konfiguriert ist, die Benutzerschnittstelle zu steuern, um weiter eine Anzeige des Ergebnisses der Bestimmung auszugeben, ob die Amplituden und/oder die Signalqualität innerhalb eines vorbestimmten Zielbereichs liegen.

11. System nach einem der vorstehenden Ansprüche, wobei die Verarbeitungseinheit auf einer mobilen Vorrichtung implementiert ist, und die Verarbeitungseinheit dazu konfiguriert ist, eine drahtlose Verbindung mit zumindest einer der Folgenden herzustellen: der Kerneinheit, der Bewegungserfassungseinheit und der PPG-Sensoreinheit.

12. System nach einem der Ansprüche 1 bis 10, wobei die Verarbeitungseinheit Teil der Kerneinheit ist, und die Kerneinheit so konfiguriert ist, dass sie an oder neben einem oberen Teil des Brustbereichs des Subjekts platziert werden kann.

13. System nach Anspruch 12, wobei die Kerneinheit auf einer mobilen Vorrichtung implementiert ist.

14. System nach Anspruch 11 oder Anspruch 13, wobei die mobile Vorrichtung ein Smartphone ist.

## Revendications

1. Système (100) pour fournir une aide à la décision de réanimation cardio-pulmonaire, RCP, le système comprenant :
une unité de détection de photopléthysmographie, PPG, (110) configurée pour déterminer un ou plusieurs signaux de PPG au niveau d'un site de mesure sur un sujet ;
une unité centrale (120) comprenant une interface utilisateur (122) ;
une unité de détection de mouvement (130) configurée pour détecter des mouvements corrélés à des compressions thoraciques pendant une thérapie par compression sur le sujet ; et
une unité de traitement (140) configurée pour :
déterminer la présence ou l'absence d'un pouls spontané sur la base des mouvements détectés corrélés à des compressions thoraciques pendant une thérapie par compression sur le sujet et des un ou plusieurs signaux de PPG ;
déterminer une recommandation à fournir sur la base de la détermination de la présence ou de l'absence d'un pouls spontané, dans lequel la recommandation est associée à une aide à la décision de RCP ;
commander l'interface utilisateur pour délivrer la recommandation déterminée ;
**caractérisé en ce que** l'unité de traitement est configurée en outre pour :
déterminer si des amplitudes et/ou une qualité de signal correspondant aux mouvements détectés corrélés à des compressions thoraciques pendant une thérapie par compression sur le sujet se situent dans une plage cible prédéterminée ;
commander l'interface utilisateur pour délivrer une instruction pour ajuster une position de l'unité de détection de mouvement ou une position de l'unité centrale si l'unité de détection de mouvement est intégrée à l'unité centrale, s'il est déterminé que les amplitudes et/ou la qualité de signal correspondant aux mouvements détectés corrélés à des compressions thoraciques pendant une thérapie par compression sur le sujet ne se situent pas dans la plage cible prédéterminée ; et
guider un utilisateur dans l'ajustement de la position de l'unité centrale et/ou de l'unité de détection de mouvement à l'aide de la lecture de l'unité de détection de mouvement.

2. Système selon la revendication 1, dans lequel l'unité de détection de mouvement fait partie de l'unité centrale.

3. Système selon la revendication 1 ou la revendication 2, dans lequel, s'il est déterminé qu'un pouls spontané est présent, la recommandation déterminée est de réaliser une vérification supplémentaire de la présence d'un pouls et/ou de suspendre l'administration d'un vasopresseur.

4. Système selon l'une quelconque des revendications précédentes, dans lequel, s'il est déterminé qu'un pouls spontané est absent, la recommandation déterminée est de poursuivre une thérapie par compression sur le sujet.

5. Système selon l'une quelconque des revendications précédentes, dans lequel l'unité de détection PPG comprend au moins un : d'un capteur de PPG alaire nasal, d'un capteur de PPG de columelle nasale, d'un capteur de PPG de conque d'oreille et d'un capteur de PPG frontal.

6. Système selon la revendication 5, dans lequel l'unité de détection PPG comprend plus d'un : d'un capteur de PPG alaire nasal, d'un capteur de PPG de columelle nasale, d'un capteur de PPG de conque d'oreille et d'un capteur de PPG frontal et l'interface utilisateur est configurée pour recevoir une entrée utilisateur indiquant au moins un capteur à activer pour déterminer les un ou plusieurs signaux PPG.

7. Système selon la revendication 5 ou la revendication 6, dans lequel l'unité de détection de PPG comprend plus d'un : d'un capteur de PPG alaire nasal, d'un capteur de PPG de columelle nasale, d'un capteur de PPG de conque d'oreille et d'un capteur de PPG frontal et dans lequel l'unité de traitement est configurée pour :
recevoir un ou plusieurs indicateurs de qualité de signal correspondant à un ou plusieurs des capteurs de PPG ; et
déterminer au moins un des un ou plusieurs capteurs de PPG à activer sur la base des un ou plusieurs indicateurs de qualité de signal reçus.

8. Système selon l'une quelconque des revendications précédentes, dans lequel l'unité de traitement est en outre configurée pour :
acquérir une valeur de température corporelle centrale du sujet ; et
commander l'interface utilisateur pour délivrer au moins un : de la température corporelle centrale détectée, d'une indication indiquant si la température corporelle centrale détectée se situe, ou non, dans une plage cible prédéterminée ou à une valeur cible prédéterminée, et d'une indication indiquant si la température corporelle centrale détectée s'approche, ou non, d'une plage cible prédéterminée ou d'une valeur cible prédéterminée.

9. Système selon l'une quelconque des revendications précédentes, dans lequel l'unité de traitement est en outre configurée pour acquérir au moins un : d'un signal d'électrocardiographie, ECG, associé au sujet et d'un signal indiquant un écoulement nasal du sujet,
dans lequel la détermination de la recommandation à fournir est en outre basée sur le au moins un signal acquis parmi un signal d'ECG et d'un signal indiquant un écoulement nasal du sujet.

10. Système selon l'une quelconque des revendications précédentes, dans lequel l'unité de traitement est en outre configurée pour commander l'interface utilisateur pour délivrer en outre une indication du résultat de la détermination pour savoir si les amplitudes et/ou la qualité de signal se situent, ou non, dans une plage cible prédéterminée.

11. Système selon l'une quelconque des revendications précédentes, dans lequel l'unité de traitement est mise en œuvre sur un dispositif mobile et l'unité de traitement est configurée pour être connectée sans fil à au moins une : de l'unité centrale, de l'unité de détection de mouvement et de l'unité de détection PPG.

12. Système selon l'une quelconque des revendications 1 à 10, dans lequel l'unité de traitement fait partie de l'unité centrale et l'unité centrale est configurée de telle sorte qu'elle puisse être placée au niveau, ou à proximité, d'une partie supérieure de la zone thoracique du sujet.

13. Système selon la revendication 12, dans lequel l'unité centrale est mise en œuvre au niveau d'un dispositif mobile.

14. Système selon la revendication 11 ou la revendication 13, dans lequel le dispositif mobile est un téléphone intelligent.
